# EUROPÄISCHE PATENTSCHRIFT

(11) **EP 2 804 529 B1**
(45) Veröffentlichungstag und Bekanntmachung des Hinweises auf die Patenterteilung: **03.07.2019**
(21) Anmeldenummer: 12790483.7
(22) Anmeldetag: 09.11.2012
(51) Int. Cl.: A61B 5/08, A61M 11/08, A61M 15/00, A61M 16/08

(54) **INHALATIONSUNTERSTÜTZUNGSVORRICHTUNG**
INHALATION SUPPORT APPARATUS
SYSTÈME D'AIDE À L'INHALATION

(30) Priorität: 20.01.2012 DE 102012200815
(43) Veröffentlichungstag der Anmeldung: 26.11.2014
(73) Patentinhaber: Kirsch, Dieter, 99947 Bad Langensalza (DE)
(72) Erfinder: KIRSCH, Anja, 99947 Bad Langensalza (DE)
(74) Vertreter: Maikowski & Ninnemann Patentanwälte Partnerschaft mbB
(86) Internationale Anmeldenummer: PCT/EP2012/072270
(87) Internationale Veröffentlichungsnummer: WO 2013/107533

(56) Entgegenhaltungen:
- EP-A1- 0 667 168
- WO-A1-01/58514
- US-A- 4 257 415
- US-A1- 2006 206 036
- US-B1- 6 578 571

## Beschreibung

### Gebiet der Erfindung

Die Erfindung betrifft eine Inhalationsunterstützungsvorrichtung für Inhalationseinrichtungen, welche ein Mundstück und einen Inhalationsgemischerzeuger zur Bereitstellung eines zu inhalierenden Inhalationsgemisches aufweisen. Die Offenbarung betrifft außerdem ein Verfahren zur Unterstützung der Inhalation eines zu inhalierenden Inhalationsgemisches mittels einer Inhalationsvorrichtung. Die Offenbarung betrifft weiterhin eine Vorrichtung für die Abnahme von Druckänderungen, deren Verwendung im medizinischen und nichtmedizinischen Bereich und ein Verfahren zum Erkennen von Atemproblemen.

Inhalationsunterstützungsvorrichtungen sind grundsätzlich aus dem Stand der Technik bekannt. EP 0 667 168 offenbart eine Inhalationsunterstützungsvorrichtung für eine ein Mundstück und einen Inhalationsgemischerzeuger zur Bereitstellung eines zu inhalierenden Inhalationsgemisches aufweisende Inhalationseinrichtung, umfassend einen Druckabnahmeschlauch, der an dem Inhalationsgemischerzeuger bzw. dem Mundstück anschließbar ist. Dafür muss jedoch an dem Inhalationsgemischerzeuger bzw. dem Mundstück eine Anschlussmöglichkeit für den Druckabnahmeschlauch vorgesehen sein. WO 01/58514 offenbart eine Inhalationsvorrichtung, die dazu gedacht ist, selektiv wirkstoffbeladene Luft bzw. Luft, die nicht mit Wirkstoff beladen ist, einem möglichen Patienten zuzuführen. Dazu weist die Inhalationsvorrichtung ein kompliziertes System an Ventilen, Klappen und Sensoren auf. Als Druckabnehmer weist die Inhalationsvorrichtung einen Sensor auf, der direkt zwischen einem Mundstück (Multi-Dose-Inhaler) (MDI) und einer Haltkammer angeordnet ist und durch den das zu inhalierende Luft-Wirkstoff- bzw. Luft-Medikamentengemisch direkt hindurchdurchströmt.

### Kurzdarstellung der Erfindung

Inhalation stellt eine effektive Methode dar, um z.B. bei Atemwegserkrankungen Wirkstoffe direkt in die betroffenen Atemwege zu bringen. Dabei wird der Wirkstoff als Bestandteil eines Inhalationsgemisches eingeatmet.

Man unterscheidet die Vernebler- oder Aerosolinhalation und die Trockeninhalation. In der Regel wird ein Aerosol des Wirkstoffes oder eine Mischung aus Dampf eines Lösungsmittels (z.B. Wasser) für den Wirkstoff und Atemluft verwendet. Bei der Trockeninhalation werden pulverisierte Wirk- und Zusatzstoffe eingeatmet. Die erfindungsgemäße Inhalationsunterstützungsvorrichtung ist sowohl für den Einsatz bei der Aerosolinhalation als auch der Trockeninhalation geeignet.

Regelmäßig ist die Inhalation dann besonders wirksam, wenn die inhalierende Person gleichmäßig und ausreichend tief ein- und ausatmet. Dabei besteht für die inhalierende Person oftmals ein Problem darin, einen entsprechenden Atemvorgang bzw. einen geeigneten Atemrhythmus aufrecht zu erhalten. Besonders problematisch kann sich eine Inhalation auch für ein Kind darstellen, für das inhalieren oftmals ein mühsamer und unerwünschter Vorgang ist.

Der Erfindung liegt die Aufgabe zugrunde, eine inhalierende Person bei der Inhalation zu unterstützen oder sie zu einem wirksamen Inhalationsvorgang bzw. Inhalationsrhythmus zu veranlassen.

Diese Aufgabe wird durch eine Inhalationsunterstützungsvorrichtung nach dem Anspruch 1 gelöst, welche zum Einsatz mit Inhalationseinrichtungen vorgesehen ist, die ein Mundstück sowie einen Inhalationsgemischerzeuger zur Bereitstellung eines zu inhalierenden Inhalationsgemisches aufweisen. Die Inhalationsunterstützungsvorrichtung weist ein Druckabnehmerzwischenstück auf, welches lösbar zwischen dem Inhalationsgemischerzeuger und dem Mundstück der Inhalationseinrichtung anordenbar ist, wobei mit dem Druckabnehmerzwischenstück Druckänderungen des Inhalationsgemisches abnehmbar sind. Außerdem ist eine Steuereinrichtung vorgesehen, welche dazu konfiguriert ist, ein auf einer Datenverarbeitungsanlage (nicht Teil der Inhalationsunterstützungsvorrichtung) ablaufendes Unterhaltungsprogramm in Abhängigkeit der mittels des Druckabnehmerzwischenstücks abgenommenen Druckänderungen anzusteuern.

### Kurzbeschreibung der Zeichungen

- Figur 1: zeigt eine Inhalationseinrichtung.
- Figur 2: zeigt die Inhalationseinrichtung aus Figur 1 in auseinandergebautem Zustand;
- Figur 3: zeigt eine erfindungsgemäße Inhalationsunterstützungsvorrichtung;
- Figur 4: zeigt eine Detailansicht der Inhalationsunterstützungsvorrichtung aus Figur 3;
- Figur 5: zeigt eine weitere Detailansicht der Vorrichtung gemäß Figur 3;
- Figur 6: zeigt eine Inhalationseinrichtung mit einer erfindungsgemäßen Inhalationsunterstützungsvorrichtung;

### Detaillierte Beschreibung der Erfindung

Die erfindungsgemäße Vorrichtung dient der Unterstützung des Inhalationsvorganges bei gängigen Inhalationseinrichtungen. Bei solchen Inhalationseinrichtungen strömt das zu inhalierende Inhalationsgemisch in der Regel von dem Inhalationsgemischerzeuger durch einen Inhalationskanal und durch das Mundstück, mittels welchem eine Person das Inhalationsgemisch einatmen kann. Die erfindungsgemäße Vorrichtung kann dabei leicht auch nachträglich in eine derartige Inhalationseinrichtung eingebaut werden, da diese oftmals zu Reinigungszwecken auseinandergebaut werden können. Darüber hinaus sind in vielen Haushalten heutzutage Datenverarbeitungsanlagen (PC, Spielekonsolen oder Ähnliches, mobile Telefone) bereits vorhanden, weshalb die erfindungsgemäße Vorrichtung problemlos eingesetzt werden kann.

Grundsätzlich werden drei unterschiedliche Inhalationssysteme unterschieden: Dosier-Aerosole (DA), Pulverinhalatoren und elektrische Verneblersysteme. Die erfindungsgemäße Inhalationsunterstützungsvorrichtung ist dabei prinzipiell für die Verwendung mit allen drei Inhalationssystemen geeignet.

In in einem Beispiel wird die erfindungsgemäße Vorrichtung zusammen mit einem Dosier-Aerosol oder einem elektrischen Verneblungssystem verwendet. In einem Vernebler wird die Inhalationslösung entweder mit Ultraschall oder mittels Druckluft über eine Düse zerstäubt. Besonders bevorzugt ist eine Ausführungsform, in der das Dosier-Aerosol ein Treibgasaerosol ist. Am meisten bevorzugt ist eine Ausführungsform, in der das Treibgasaerosol zusätzlich einen Abstandshalter (Spacer) aufweist.

In in einem Beispiel wird die erfindungsgemäße Vorrichtung zusammen mit einem Pulverinhalator verwendet.

Im Betrieb der Vorrichtung zusammen mit einer Inhalationseinrichtung und einer Datenverarbeitungsanlage ist die Datenverarbeitungsanlage der inhalierenden Person zugänglich aufgestellt. Auf der Datenverarbeitungsanlage läuft ein Unterhaltungsprogramm ab. Da das Unterhaltungsprogramm durch die Druckänderungen des Inhalationsgemisches gesteuert wird, kann die inhalierende Person durch kontrolliertes Ein- und Ausatmen über die so hervorgerufenen Druckänderungen im Inhalationsgemisch auf das Unterhaltungsprogramm Einfluss nehmen. Denkbar ist diesbezüglich insbesondere ein Unterhaltungsprogramm in der Art eines Computerspiels, bei dem bestimmte Spiel-Aktionen über die abgenommenen Druckänderungen steuerbar sind. Dadurch kann die Person bei der Inhalation unterstützt werden. Insbesondere kann durch eine geeignete Ausgestaltung des Unterhaltungsprogramms, zum Beispiel durch in bestimmten Zeitabständen erzeugte Anreize zur Einflussnahme zum Beispiel auf eine Spiel-Aktion, eine inhalierende Person gezielt zum Einbeziehungsweise Ausatmen angeregt werden. Auf diese Weise wird der Inhalationsvorgang quasi spielerisch unterstützt.

Die genannte Abnahme von Druckänderungen mittels des Druckabnehmerzwischenstücks bezeichnet im vorliegenden Zusammenhang nicht notwendigerweise bereits eine Wandlung der Druckänderung in elektrische Messsignale, wie dies beispielsweise mit einem Druckmesswandler beziehungsweise einem Drucksensor erfolgen kann. Vorteilhaft ist vielmehr, wenn der (momentan) in dem Inhalationsgemisch wirkende Druck (insbesondere der im Mundstück oder in einem Inhalationskanal zur Durchleitung des Inhalationsgemisches vom Inhalationsgemischerzeuger zum Mundstück herrschende Druck) oder die Änderung dieses Druckes über eine kommunizierende, das heißt in Druckverbindung stehende, Druckleitung zu einem Druckmesswandler weitergeführt wird. Insbesondere werden die abgenommenen Druckänderungen der Steuereinrichtung derart zugeführt, dass die Druckänderungen auch in der Steuereinrichtung wirken (bzw. auf einen in/an der Steuereinrichtung vorgesehenen Messwandler wirken). Eine Umwandlung in elektrische Messsignale erfolgt in diesem Fall in der, insbesondere beabstandet zum Druckabnehmerzwischenstück angeordneten, Steuereinrichtung.

Die Steuereinrichtung umfasst vorzugsweise einen konventionellen Prozessor, den Druckmesswandler und eine Software zur Steuerung der Komponenten und Signalumwandlung. Der Druckmesswandler ist vorzugsweise ein Drucksensor. Besonders bevorzugt ist dabei ein Differenzdrucksensor, der eine hohe Empfindlichkeit aufweist.

Vorzugsweise sind die abgenommenen Druckänderungen mittels der Steuereinrichtung in Steuersignale zur Ansteuerung des auf der Datenverarbeitungsanlage ablaufenden Unterhaltungsprogrammes umwandelbar. Die Steuereinrichtung wirkt hierzu über eine Kommunikationsverbindung, beispielsweise eine serielle oder parallele Schnittstelle, USB-Verbindung, Mini-USB-Verbindung oder WLAN-Verbindung oder Bluetooth-Verbindung, mit der Datenverarbeitungsanlage zusammen.

Eine besonders bevorzugte Ausgestaltung ergibt sich dadurch, dass das lösbar angeordnete Druckabnehmerzwischenstück insbesondere zwischensteckbar zwischen dem Inhalationsgemischerzeuger und dem Mundstück anordenbar ist.

Dadurch ist es möglich, die erfindungsgemäße Vorrichtung nur bei Bedarf in einer gängigen Inhalationseinrichtung anzuordnen. Die Inhalationsunterstützungsvorrichtung kann jedoch auch wieder von dem Inhalator getrennt werden, so dass eine komfortable und gründliche Reinigung, insbesondere auch der Verbindungsstellen, zu dem Mundstück und dem Inhalationsgemischerzeuger möglich ist.

Vorteilhaft ist ferner, wenn das Druckabnehmerzwischenstück einen Zugangssteckabschnitt zum Zusammenstecken mit einem zugeordneten Steckabschnitt des Inhalationsgemischerzeugers sowie einen Ausgangssteckabschnitt zum Zusammenstecken mit einem zugeordneten Steckabschnitt des Mundstücks aufweist. Bei gängigen Inhalationseinrichtungen ist oftmals das Mundstück aufsteckbar ausgebildet, um beispielsweise eine Reinigung auf einfache Weise zu ermöglichen. Der Zugangssteckabschnitt und der Ausgangssteckabschnitt sind dabei vorzugsweise an die entsprechenden Steckabschnitte gängiger Inhalationseinrichtungen angepasst ausgebildet.

Zur weiteren Ausgestaltung der erfindungsgemäßen Vorrichtung ist vorgesehen, dass das Druckabnehmerzwischenstück einen Durchströmkanal zur Durchleitung des Inhalationsgemisches, insbesondere von dem Inhalationsgemischerzeuger zu dem Mundstück, und einen mit dem Durchströmkanal druckkommunizierenden Druckabnahmezugang aufweist. Der Druckabnahmezugang wirkt also mit dem Durchströmkanal derart zusammen, dass der im Durchströmkanal wirkende Druck des Inhalationsgemisches über den Druckabnahmezugang übertragen beziehungsweise weitergegeben werden kann, oder dass der Druck des Inhalationsgemisches auch in dem Druckabnahmezugang beziehungsweise in einem mit diesem verbundenen Druckabnahmeraum beziehungsweise einer mit diesem verbundenen Druckabnahmeleitung herrscht.

Denkbar ist aber auch, dass das Druckabnehmerzwischenstück einen Druckmesswandler aufweist, welcher mit dem Durchströmkanal derart zusammenwirkt, dass der Druck in dem Durchströmkanal messbar ist.

Vorteilhaft ist insbesondere, wenn der Druckabnahmezugang in den Durchströmkanal mündet. Dabei weist der Durchströmkanal insbesondere eine wirksame Querschnittsfläche (Durchströmquerschnitt) für die Strömung des Inhalationsgemisches auf, und der Druckabnahmezugang weist eine wirksame Querschnittsfläche (Druckabnahmequerschnitt) auf, wobei der Druckabnahmequerschnitt wesentlich kleiner als der Durchströmquerschnitt ist, insbesondere das Verhältnis von Druckabnahmequerschnitt zu Durchströmquerschnitt im Bereich von 1:100 bis 1:10 liegt. Dadurch kann vermieden werden, dass durch den Druckabnahmezugang ein merklicher Druckabfall im Durchströmkanal hervorgerufen wird.

Bevorzugt ist eine Ausführungsform, in der das Verhältnis von Druckabnahmequerschnitt zu Durchströmquerschnitt ein Verhältnis von 1:100, 1:90, 1:80, 1:70, 1:60, 1:50, 1:40, 1:30, 1:20 oder 1:10 aufweist. Besonders bevorzugt ist eine Ausführungsform, in der das Verhältnis von Druckabnahmequerschnitt zu Durchströmquerschnitt ein Verhältnis von 1:100, 1:90, 1:80, 1:70, 1:60 oder 1:50 aufweist. Ebenso bevorzugt ist eine Ausführungsform, in der das Verhältnis von Druckabnahmequerschnitt zu Durchströmquerschnitt im Bereich von 1:100 bis 1:50 liegt. Weiterhin bevorzugt ist eine Ausführungsform, in der das Verhältnis von Druckabnahmequerschnitt zu Durchströmquerschnitt ein Verhältnis von 1:100, 1:90, 1:80 oder 1:70 aufweist. Weiterhin bevorzugt ist eine Ausführungsform, in der das Verhältnis von Druckabnahmequerschnitt zu Durchströmquerschnitt im Bereich von 1:100 bis 1:70 liegt. Am meisten bevorzugt ist eine Ausführungsform, in der das Verhältnis von Druckabnahmequerschnitt zu Durchströmquerschnitt ein Verhältnis von 1:100 oder 1:90 aufweist. Ebenso bevorzugt ist eine Ausführungsform, in der das Verhältnis von Druckabnahmequerschnitt zu Durchströmquerschnitt im Bereich von 1:100 bis 1:90 liegt.

Der Druckabnahmezugang mündet insbesondere in einer das Inhalationsgemisch leitenden Innenwandung des Durchströmkanals.

Vorteilhaft ist außerdem eine Ausgestaltung der Vorrichtung, bei der das Druckabnehmerzwischenstück über eine flexible Druckabnahmeleitung zur Weiterleitung des abgenommenen Druckes beziehungsweise der abgenommenen Druckänderungen mit der Steuereinrichtung druckverbunden ist. Dadurch wird eine komfortablere Benutzung der erfindungsgemäßen Vorrichtung während der Inhalation und der gleichzeitigen Einflussnahme auf ein Computerprogramm an der angeschlossenen Datenverarbeitungsanlage ermöglicht.

Die Druckabnahmeleitung ist insofern zwischen dem Druckabnehmerzwischenstück und der Steuereinrichtung angeordnet und verbindet diese. Insbesondere ist die Druckabnahmeleitung derart mit dem Druckabnahmezugang druckverbunden, dass der abgenommene Druck durch die Druckabnahmeleitung weiter gegeben wird. Die Steuereinrichtung weist insbesondere einen Druckzugang auf, über welche der Steuereinrichtung Druckänderungen zuführbar sind. Die Druckabnahmeleitung kann mit diesem Druckzugang druckverbunden werden.

Zur weiteren Ausgestaltung ist in der Druckabnahmeleitung ein Filterelement vorgesehen, mittels welchem insbesondere in dem Inhalationsgemisch enthaltene Flüssigkeitströpfchen und/oder Verunreinigungen, wie zum Beispiel krankheitserregende Mirkoorganismen, aufgefangen werden können. Das Filterelement ist insbesondere zwischen dem Druckabnehmerzwischenstück und der Steuereinrichtung in der Druckabnahmeleitung angeordnet. So kann vermieden werden, dass Flüssigkeit oder Verunreinigungen(zum Beispiel aus dem Inhalationsgemisch oder aus der ausgeatmeten Luft) in die Steuereinrichtung gelangen und dort zu Verunreinigung oder Schäden führen. Denkbar ist auch, dass das Filterelement in oder an dem Druckabnehmerzwischenstück oder in oder an der Steuereinrichtung angeordnet ist.

Geeignete Filterelemente weisen eine Filterkapsel, die ein Filtermaterial einschließt, sowie einen Zugang und einen Ausgang derart auf, dass ein gasförmiges Medium durch den Zugang zu dem Filtermedium und nach Durchtritt durch das Filtermedium aus dem Ausgang ausströmen kann.

Zur Rückhaltung von Mikroorganismen eignen sich zum Beispiel konventionelle Sterilfilter, wie Bakterienfilter oder Virenfilter, die wie zuvor beschrieben aufgebaut sind.

Als Filtermedien für die Rückhaltung von Feuchtigkeit oder Flüssigkeitstropfen sind Feuchtigkeitsadsorber, wie zum Beispiel handelsübliche Silikagele oder Zeolithe, geeignet.

Weitere geeignete Filtermedien sind Membranen mit einer sehr engen Porenweite, die sowohl Mikroorganismen als auch Flüssigkeitstropfen zurückhalten können. Vorzugsweise bestehen solche Membranen aus Teflon.

Wahlweise können in der Vorrichtung auch zwei oder mehr Filterelemente hintereinander angeordnet werden, wie zum Beispiel ein Bakterienfilter und ein Filterelement, das ein Adsorbermaterial enthält.

Eine besonders bevorzugte Ausgestaltung der Inhalationsunterstützungsvorrichtung ergibt sich dadurch, dass das Druckabnehmerzwischenstück elektronikfrei, das heißt ohne elektronische Bauteile beziehungsweise frei von stromführenden oder spannungsführenden Bauteilen ausgebildet ist.

Insbesondere ist bei dieser Ausgestaltung das Druckabnehmerzwischenstück frei von Messelektronik. Der erforderliche Druckmesswandler ist ebenfalls nicht in dem Druckabnehmerzwischenstück angeordnet. Diese elektronikfreien Ausführungen des Druckabnehmerzwischenstücks haben den Vorteil, dass eine Reinigung auch mit Flüssigkeit leicht möglich ist und Beschädigungen beispielsweise der Messelektronik durch Flüssigkeit vermieden werden können. Das Druckabnehmerzwischenstück kann beispielsweise problemlos unter fließendem Wasser gereinigt werden. Zur Reinigung können Inhalationseinrichtungen oftmals auseinandergebaut werden, insbesondere das Mundstück vom Inhalationsgemischerzeuger getrennt werden. Diejenigen Teile, welche mit Feuchtigkeit beziehungsweise mit Inhalationsgemisch beziehungsweise mit Atemluft in Berührung kommen, können dann einzeln und gründlich gereinigt werden.

Um die abgenommenen Druckänderungen in elektrische Messsignale umzuwandeln, weist die Steuereinrichtung vorzugsweise einen Druckmesswandler auf. Dieser Druckmesswandler ist insbesondere nicht in oder an dem Druckabnehmerzwischenstück, sondern in oder an der Steuereinrichtung vorgesehen. Insbesondere weist die Steuereinrichtung einen Druckzugang auf, über welchen der abgenommene Druck oder die abgenommenen Druckänderungen dem Druckmesswandler der Steuereinrichtung zugeführt werden können.

Vorteilhaft ist eine Ausgestaltung der Inhalationsunterstützungsvorrichtung, bei welcher die Steuereinrichtung ein Gehäuse aufweist, wobei das Gehäuse einen mit dem Gehäuse verbundenen Steckverbinder zur Herstellung einer Kommunikationsverbindung mit der Datenverarbeitungsanlage aufweist. Der Steckverbinder ist mit dem Gehäuse vorzugsweise starr verbunden. Dieser Steckverbinder kann insbesondere als USB-Steckerteil ausgebildet sein und derart in das Gehäuse der Steuereinrichtung integriert sein beziehungsweise mit dem Gehäuse der Steuereinrichtung starr verbunden sein, dass das Gehäuse insgesamt mit dem USB-Steckerteil an einen USB-Eingang der Datenverarbeitungsanlage angesteckt werden kann. Dies ermöglicht es auf komfortable Weise, die Inhalationsunterstützungsvorrichtung zum Betrieb an die Datenverarbeitungsanlage anzuschließen.

In dem Gehäuse der Steuereinrichtung ist vorzugsweise der Druckmesswandler, insbesondere auch sämtliche erforderliche Steuerelektronik, eingeschlossen. Das Gehäuse weist vorzugsweise einen Druckzugangsabschnitt auf, mit welchem beispielsweise die Druckabnahmeleitung verbindbar ist, um die Druckänderungen an die Steuereinrichtung beziehungsweise an einen ebenfalls im Gehäuse untergebrachten Druckmesswandler weiterzuleiten.

Eine besonders bevorzugte Ausgestaltung der erfindungsgemäßen Vorrichtung ergibt sich dadurch, dass das Unterhaltungsprogramm in der Steuereinrichtung hinterlegt ist beziehungsweise dort hinterlegbar ist. Hierzu kann die Steuereinrichtung eine Speichereinrichtung, beispielsweise Flash-Memory, aufweisen. Zum Betrieb kann die Steuereinrichtung mit der Datenverarbeitungsanlage verbunden werden und das Unterhaltungsprogramm dann auf der Datenverarbeitungsanlage ausgeführt werden. Die so ausgestaltete Inhalationsunterstützungsvorrichtung kann damit ohne Weiteres in der Art eines "Plug and Play" Gerätes in Betrieb genommen werden.

Vorteilhaft kann aber auch sein, wenn das Unterhaltungsprogramm in der Datenverarbeitungsanlage hinterlegt ist und auch dort abläuft. So können beispielsweise für die erfindungsgemäße Vorrichtung verschiedene Unterhaltungsprogramme bereitgestellt werden, unter welchen die inhalierende Person auswählen kann. So kann eine abwechslungsreiche Inhalationsunterstützung ermöglicht werden.

Die gestellte Aufgabe wird auch gelöst durch eine Inhalationsvorrichtung mit einem Inhalationsgemischerzeuger zur Bereitstellung eines zu inhalierenden Inhalationsgemisches und mit einem Mundstück, über welches das Inhalationsgemisch eingeatmet werden kann, wobei eine Inhalationsunterstützungsvorrichtung der vorstehend beschriebenen Art vorgesehen ist, deren Druckabnehmerzwischenstück zwischen dem Inhalationsgemischerzeuger und dem Mundstück angeordnet ist. Dabei ist insbesondere das Druckabnehmerzwischenstück derart angeordnet, dass im Betrieb der Inhalationsvorrichtung das Inhalationsgemisch durch das Druckabnehmerzwischenstück strömt.

Die erfindungsgemäße Inhalationsvorrichtung ist beispielsweise geeignet zur Unterstützung der Inhalation eines zu inhalierenden Inhalationsgemisches mittels einer Inhalationsvorrichtung vorgeschlagen. Bei diesem Verfahren erfolgt eine Abnahme einer Druckänderung des zu inhalierenden Inhalationsgemisches, eine Umwandlung der Druckänderung in Steuersignale zur Ansteuerung einer Datenverarbeitungsanlage und ein Ansteuern eines auf der Datenverarbeitungsanlage ablaufenden Unterhaltungsprogramms mittels der Steuersignale in Abhängigkeit der abgenommenen Druckänderung. Nach der Abnahme der Druckänderung erfolgt insbesondere eine Zuführung dieser Drückänderung zu einer Steuereinrichtung zur Umwandlung in Steuersignale.

Dadurch kann die inhalierende Person dazu veranlasst werden, wie bereits vorstehend beschrieben, durch entsprechend kontrolliertes Ein- und Ausatmen Druckänderungen im Inhalationsgemisch hervorzurufen und so auf das Unterhaltungsprogramm Einfluss zu nehmen. Denkbar ist insbesondere, dass das Unterhaltungsprogramm durch Abgabe geeigneter Reizsignale (zum Beispiel Bilddarstellungen, Tonabgabe, ...) in auf vorgebbare Weise zeitlich aufeinanderfolgenden Zeitpunkten eine Anregung zur Auslösung einer bestimmten Spiel-Aktion gibt. Diese Spiel-Aktion kann dann bei dem Unterhaltungsprogramm beispielsweise durch gezieltes Ein- und Ausatmen herbeigeführt werden. Dadurch kann beispielsweise ein aus therapeutischen Gründen erwünschter oder besonders günstiger Inhalationsrhythmus vorgegeben werden.

Die erfindungsgemäße Inhalationsvorrichtung ist besonders geeignet für den Einsatz in der Therapie und/oder der Linderung von Krankheiten.

Dementsprechend betrifft eine weitere Ausführungsform der Erfindung auch die erfindungsgemäße Inhalationsunterstützungsvorrichtung zur Verwendung in der Behandlung und/oder Linderung von Krankheiten.

Die erfindungsgemäße Vorrichtung eignet sich besonders zur Verwendung in der Therapie und/oder Linderung solcher Krankheiten, die mittels Inhalation behandelbar sind. Mit Hilfe der erfindungsgemäßen Inhalationsunterstützungsvorrichtung kann dabei eine besonders gleichmäßige und kontinuierliche Dosierung der für die jeweilige Erkrankung benötigten Medikamente erreicht werden.

Krankheiten, die durch Inhalation behandelbar sind, sind zum Beispiel Erkrankungen der Bronchien und des Nasen-Rachen-Raumes, chronische oder akute Atemwegserkrankungen sowie schwere virale oder bakterielle Infektionen der Atemwege.

In einer bevorzugten Ausführungsform dient die erfindungsgemäßen Inhalationsunterstützungsvorrichtung zur Verwendung in beispielsweise der Behandlung von Bronchitis, Asthma, Zöliakie, COPD (Chronisch-obstruktive Lungenerkrankung), allergischem Schnupfen (Rhinitis allergica), Lungenentzündung, Pilzerkrankungen der Lunge, Lungentuberkulose oder schwerem akuten respiratorischen Syndrom (Sars).

In einer weiteren bevorzugten Ausführungsform dient die erfindungsgemäßen Inhalationsunterstützungsvorrichtung zur Verwendung in der Linderung von Symptomen von Stoffwechselerkrankungen, insbesondere durch einen genetischen Defekt verursachte Stoffwechselerkrankungen.

Ein Beispiel für solch eine durch einen genetischen ist Mukoviszidose, auch cystische Fibrose genannt. Mukoviszidose wird durch einen Gendefekt auf dem menschlichen Chromosom 7 verursacht. Symptome der Erkrankung sind dauernder Husten, eventuell häufige Lungenentzündungen und Atemnot-Situationen, Verdauungsstörungen mit Bauchschmerzen, Wachstumsstörungen und Untergewicht. Die Mukoviszidose ist trotz intensiver Bemühungen der medizinischen und pharmazeutischen Forschung bislang nicht heilbar. Die Behandlung dieser Erkrankung beschränkt sich deshalb auf die stete Verbesserung der konventionellen Behandlungsmethoden sowie die Therapie von häufigen Begleiterkrankungen, wie etwa Infektionen der Atemwege oder Lungenentzündungen.

Die Inhalation ist bei der therapeutischen Behandlung von Mukoviszidose-Patienten von zunehmender Bedeutung. Während anfangs die Inhalationstherapie hauptsächlich dazu diente, den zähen Schleim in den Atemwegen zu lösen, damit ihn die Betroffenen leichter abhusten können, nutzt man die Inhalationstherapie heute mehr und mehr dazu, Medikamente zu verabreichen. Lungenwirksame Medikamente können so auf direktem Wege an den Wirkort gelangen. Entsprechend der unterschiedlichen Therapieziele gibt es auch verschiedene inhalative Substanzen, die Mukoviszidose-Patienten inhalieren können. Die wichtigsten inhalativen Substanzen sind schleimlösende Substanzen, bronchienerweiternde Substanzen, entzündungshemmende Substanzen und Antibiotika zur Behandlung bakterieller Infektionen der Lunge.

Eine besonders bevorzugte Ausführungsform der Erfindung betrifft deshalb die Inhalationsunterstützungsvorrichtung zur Verwendung in Verfahren zur Behandlung von Mukoviszidose. Bei der Mukoviszidosebehandlung ist die Verwendung der erfindungsgemäßen Vorrichtung dann besonders vorteilhaft, wenn die inhalierende Person ein Kind ist und das Kind durch die Vorrichtung bei der Inhalation unterstützt oder zu einem wirksamen Inhalationsvorgang bzw. Inhalationsrhythmus veranlasst wird.

Neuerdings werden auch für die Behandlung andere Krankheiten Medikamente entwickelt, die mittels Inhalation verabreicht werden. Ein Beispiel hierfür ist die Verabreichung von inhalierbarem Insulin zur Behandlung von Typ-1- und Typ-2-Diabetes. Eine weitere bevorzugte Ausführungsform der Erfindung betrifft deshalb die Inhalationsunterstützungsvorrichtung zur Verwendung in Verfahren zur Behandlung von Typ-1- und Typ-2-Diabetes.

Als Datenverarbeitungsanlage eignet sich in dem offenbarten Verfahren jeder herkömmliche Computer, wie zum Beispiel ein Desktop-PC, ein Laptop-PC oder ein Tablet-PC, aber auch jede in einer medizinischen Vorrichtung oder Diagnosegerät integrierte Datenverarbeitungsanlage. Ebenso geeignet als Datenverarbeitungsanlage ist ein mobiles Telefon, wie zum Beispiel ein Smart-Phone.

Weitere Einzelheiten und vorteilhafte Ausgestaltungen der Erfindung sind der nachfolgenden Beschreibung zu entnehmen, anhand derer die in den Figuren dargestellten Ausführungsformen der Erfindung näher beschrieben und erläutert sind.

Aus Gründen der Übersichtlichkeit werden im Folgenden für gleiche oder einander entsprechende Bauteile in allen Figuren dieselben Bezugszeichen verwendet.

### 1. Inhalationsunterstützungsvorrichtung

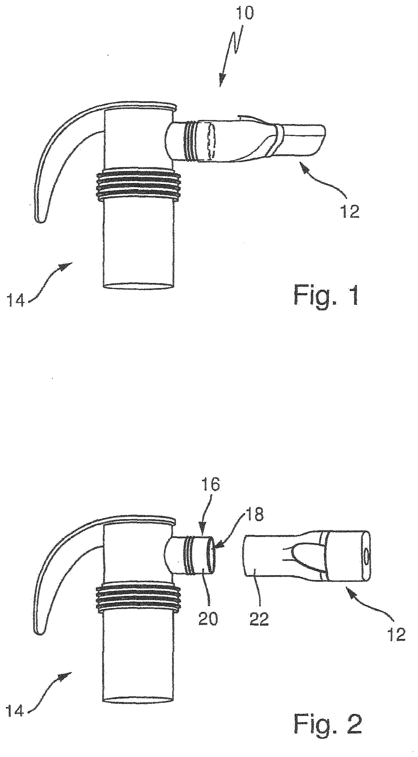

Die Figur 1 zeigt eine Inhalationseinrichtung 10, welche ein Mundstück 12 und einen Inhalationsgemischerzeuger 14 umfasst. Der Inhalationsgemischerzeuger 14 dient dazu, ein zu inhalierendes Inhalationsgemisch (beispielsweise ein Aerosol eines insbesondere flüssigen Wirkstoffes) bereitzustellen. Hierzu kann der Inhalationsgemischerzeuger 14 einen nicht näher dargestellten Flüssigkeitstank für den Wirkstoff aufweisen. Außerdem kann ein nicht dargestellter Vernebler vorgesehen sein, mittels welchem beispielsweise durch Druckluft der flüssige Wirkstoff in ein Aerosol überführt werden kann. Zu diesem Zweck kann an dem Inhalationsgemischerzeuger 14 beispielsweise über einen nicht näher dargestellten Druckluftanschluss ein Kompressor zur Bereitstellung der erforderlichen Druckluft für den Vernebler vorgesehen sein.

Über das Mundstück 12 kann die inhalierende Person das Inhalationsgemisch einatmen.

Wie aus der Figur 2 erkennbar, ist die Inhalationseinrichtung 10 insbesondere zu Reinigungszwecken zerlegbar. Dabei kann das Mundstück 12 von dem Inhalationsgemischerzeuger 14 abgezogen werden.

Der Inhalationsgemischerzeuger 14 weist einen Stutzen 16 auf, auf welchen das Mundstück 12 lösbar aufsteckbar ist. Der Stutzen 16 ist als Hohlrohrabschnitt ausgebildet und umschließt einen Inhalationskanal 18, durch welchen das bereitgestellte Inhalationsgemisch strömt.

Um das Mundstück 12 auf den Stutzen 16 aufstecken zu können, weist der Stutzen 16 einen ersten Steckabschnitt 20 auf, welcher zum Zusammenstecken mit einem entsprechend ausgebildeten zugeordneten zweiten Steckabschnitt 22 des Mundstücks 12 ausgebildet ist.

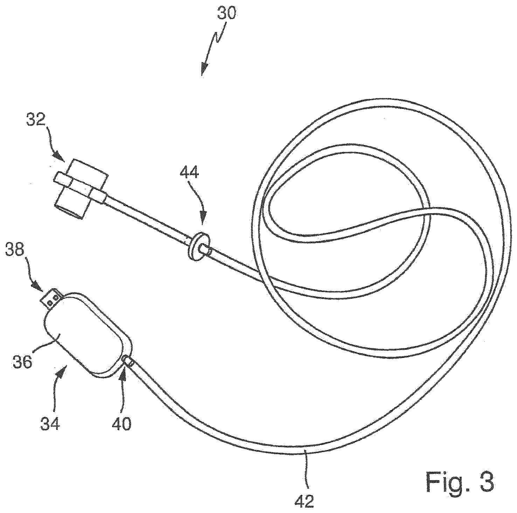

In der Figur 3 ist eine erfindungsgemäße Inhalationsunterstützungsvorrichtung 30 dargestellt, welche mit der in den Figuren 1 und 2 dargestellten Inhalationseinrichtung verwendet werden kann. Die Inhalationsunterstützungsvorrichtung weist ein Druckabnehmerzwischenstück 32 sowie eine Steuereinrichtung 34 auf.

Die Steuereinrichtung 34 hat ein Gehäuse 36, welches deren elektronische Bauteile einschließen. Das Gehäuse 34 weist einen aus dem Gehäuse 34 hervorragenden, mit dem Gehäuse 34 starr verbundenen Steckverbinder 38 zur Herstellung einer Kommunikationsverbindung mit einer nicht dargestellten Datenverarbeitungsanlage auf. Der Steckverbinder 38 ist als USB-Steckerteil zum Einstecken in eine USB-Steckbuchse der Datenverarbeitungsanlage ausgebildet.

Das Gehäuse 36 der Steuereinrichtung 34 weist ferner einen Druckzugang 40 auf. An den Druckzugang 40 ist eine flexible Druckabnahmeleitung 42 zur Weitergabe eines in ihr anstehenden Drucks beziehungsweise in ihr wirkenden Druckänderungen angeschlossen. Die Steuereinrichtung 34 umfasst einen nicht näher dargestellten Druckmesswandler zur Umwandlung der über die Druckabnahmeleitung 42 zugeführten Druckänderungen in elektrische Messsignale auf.

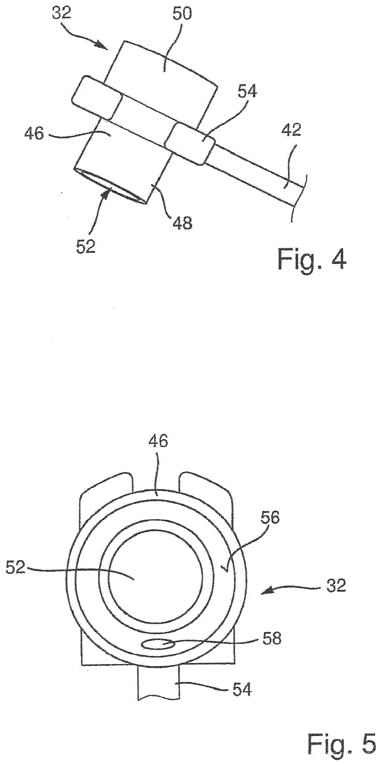

Die Druckabnahmeleitung 42 ist andererseits in einer in den Figuren 4 und 5 näher erläuterten Art mit dem Druckabnehmerzwischenstück 32 druckverbunden, so dass mittels des Druckabnehmerzwischenstückes 32 eine Druckänderung abnehmbar und über die Druckabnahmeleitung 42 an die Steuereinrichtung 34 weiterleitbar sind.

In der Druckabnahmeleitung 42 ist ein Filterelement 44 angeordnet. Dieses Filterelement 44 weist eine Filterkapsel auf, welche ein Filtermaterial einschließt. Die Filterkapsel weist einen Zugang und einen Ausgang derart auf, dass ein gasförmiges Medium durch den Zugang zu dem Filtermedium und nach Durchtritt durch das Filtermedium aus dem Ausgang ausströmen kann. Das Filtermedium ist insbesondere derart ausgebildet, dass beim Durchtritt eines gasförmigen Mediums mit in der Art eines Aerosols gelösten Flüssigkeitströpfchen die Flüssigkeitströpfchen in dem Filtermedium hängen bleiben und im Wesentlichen nur die gasförmigen Bestandteile durchtreten.

Die Figur 4 zeigt eine Detailansicht des Druckabnehmerzwischenstückes 32. Das Druckabnehmerzwischenstück 32 hat einen in der Art eines Hohlzylinders ausgebildeten Grundkörper 46, welcher einen Zugangssteckabschnitt 48 sowie einen Ausgangssteckabschnitt 50 aufweist. Mittels des Zugangssteckabschnittes 48 kann das Druckabnehmerzwischenstück 32 auf den Steckabschnitt 20 des Inhalationsgemischerzeugers 14 aufgesteckt werden. Über den Ausgangssteckabschnitt 50 ist es möglich, das Druckabnehmerzwischenstück 32 mit dem entsprechenden Steckabschnitt 22 des Mundstücks 12 zusammenzustecken. Die so entstehende Steckverbindung ist lösbar und kann beispielsweise zu Reinigungswecken leicht wieder auseinander gezogen werden.

Der hohlzylindrische Grundkörper 46 umschließt einen axial in dem Grundkörper 46 verlaufenden Durchströmkanal 52, welcher in der Figur 5 näher dargestellt ist. Wird das Druckabnehmerzwischenstück 32 wie erläutert mit dem Inhalationsgemischerzeuger 14 und dem Mundstück 12 zusammengesteckt, so kann das Inhalationsgemisch von dem Inhalationsgemischerzeuger 14 durch den Inhalationskanal 18 und durch den Durchströmkanal 52 zu dem Mundstück 12 strömen.

Der Grundkörper 46 weist ferner einen Druckabnahmezugang 54 auf, welcher durch Wandungen des in der Art eines Hohlzylinders ausgebildeten Grundkörpers 46 in den Durchströmkanal 52 mündet.

In der Figur 5 ist das Druckabnehmerzwischenstück 32 in einer Darstellung mit Blickrichtung in den axial in dem Grundkörper 46 verlaufenden Durchströmkanal 52 dargestellt. In der erkennbaren Wandung 56 des Grundkörpers 46 ist eine Mündung 58 des Druckabnehmerzugangs 54 angedeutet. Erkennbar weist der Durchströmkanal 52 einen Durchströmquerschnitt auf, welcher wesentlich größer ist als der Mündungsquerschnitt (Druckabnehmerquerschnitt) der Mündung 58 des Druckabnahmezugangs 54 (Flächenverhältnis cirka 10:1).

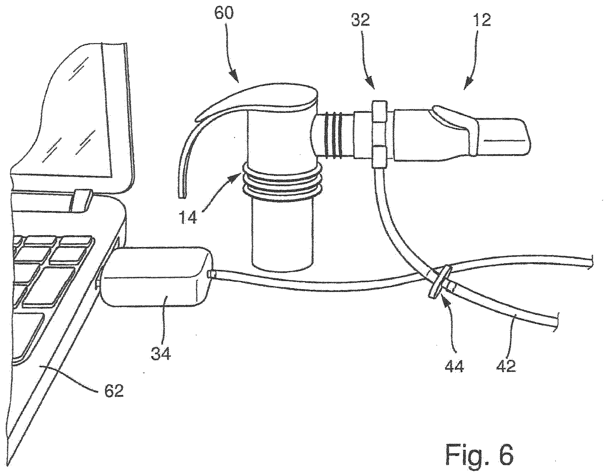

In der Figur 6 ist eine Inhalationseinrichtung 60 dargestellt, bei welcher zwischen Inhalationsgemischerzeuger 14 und Mundstück 12 das Druckabnehmerzwischenstück 32 der in der Figur 3 dargestellten Inhalationsunterstützungsvorrichtung 30 gesteckt ist.

Über die Druckabnahmeleitung 42 ist das Druckabnehmerzwischenstück 32 mit der Steuereinrichtung 34 druckverbunden. So können die bei einem Inhalationsvorgang auftretenden Druckänderungen im Inhalationskanal (vergleiche Bezugszeichen 18 in Figur 2) beziehungsweise im Durchströmkanal (Bezugszeichen 52 in den Figuren 4 und 5) von dem Druckabnehmerzwischenstück 32 an die Steuereinrichtung 34 weitergegeben werden beziehungsweise sind dieser zuführbar.

Die Steuereinrichtung 34 ist mit dem zu Figur 3 erläuterten Steckverbinder 38 an einen entsprechenden, nicht dargestellten Steckeingang einer Datenverarbeitungsanlage (hier: Notebook- PC) zur Ausbildung einer Kommunikationsverbindung zwischen der Steuereinrichtung 34 und der Datenverarbeitungsanlage 62 angeschlossen.

Auf der Datenverarbeitungsanlage 62 läuft beispielsweise ein Computerspiel ab, dessen Programmcode in der Steuereinrichtung 34 hinterlegt ist und über die genannte Kommunikationsverbindung auf die Datenverarbeitungsanlage 62 übertragbar ist. Dieses Computerspiel ist in Abhängigkeit der mittels des Druckabnehmerzwischenstücks 32 abgenommenen Druckänderungen des Inhalationsgemisches (im Durchströmkanal 52) steuerbar. Hierzu sind die abgenommenen Druckänderungen über die Druckabnahmeleitung 42 der Steuereinrichtung 34 (über den Druckabnahmezugang 54, vergleiche Figuren 4 und 5) zuführbar. Die Steuereinrichtung 34 weist einen Druckmesswandler (nicht dargestellt) auf, mittels welchem die zugeführten Druckänderungen in elektrische Messsignale umwandelbar sind. Aus den elektrischen Messsignalen sind in der Steuereinrichtung 34 Steuersignale zur Ansteuerung des auf der Datenverarbeitungsanlage 62 ablaufenden Computerspiels erzeugbar.

Die in der Steuereinrichtung (34) erzeugten elektrischen Messsignale können aber auch dazu genutzt werden, um mittels eines auf der Datenverarbeitungsanlage (62) gespeicherten geeigneten Computerprogramms Messdaten zu erzeugen, die über den Inhalationsprozess Auskunft geben. So können zum Beispiel Parameter, wie Dauer der Inhalation, Atmungsfrequenz, mögliche Unterbrechungen oder Pausen bei der Atmung aufgezeichnet werden und daraus Erkenntnisse über den Verlauf der Inhalation gewonnen werden. Mit Hilfe der so gewonnenen Messdaten kann der Inhalationsprozess für den jeweiligen Patienten individuell weiter optimiert werden.

## Patentansprüche

1. Inhalationsunterstützungsvorrichtung (30) für eine ein Mundstück(12) und einen Inhalationsgemischerzeuger (14) zur Bereitstellung eines zu inhalierenden Inhalationsgemisches aufweisende Inhalationseinrichtung (10),
umfassend
- ein Druckabnehmerzwischenstück (32), welches zwischen dem Inhalationsgemischerzeuger (14) und dem Mundstück (12) anordenbar ist,
wobei das Druckabnehmerzwischenstück (32) frei von elektronischen Bauteilenden oder frei von stromführenden oder spannungsführenden Bauteilen ausgebildet ist, und mit dem Druckabnehmerzwischenstück (32) Druckänderungen des Inhalationsgemisches abnehmbar sind,
- sowie eine Steuereinrichtung (34) zur Ansteuerung eines auf einer Datenverarbeitungsanlage (62) ablaufenden Unterhaltungsprogramms in der Art eines Computerspiels, wobei die Steuereinrichtung (34) zur Ansteuerung des Unterhaltungsprogramms in der Art eines Computerspiels in Abhängigkeit der Druckänderungen konfiguriert ist;
**dadurch gekennzeichnet, dass** das Druckabnehmerzwischenstück (32) lösbar zwischen dem Inhalationsgemischerzeuger (14) und dem Mundstück (12) anordenbar ist, insbesondere zwischensteckbar ist.

2. Vorrichtung (30) nach Anspruch 1, **dadurch gekennzeichnet, dass** die Steuereinrichtung (34) mit dem Druckabnehmerzwischenstück (32) zur Zuführung der abgenommenen Druckänderungen zur Steuereinrichtung (34) druckverbunden ist.

3. Vorrichtung (30) nach Anspruch 1 oder 2, **dadurch gekennzeichnet, dass** das Druckabnehmerzwischenstück (32) einen Durchströmkanal (52) zur Durchleitung des Inhalationsgemisches und einen mit dem Durchströmkanal (52) druckkommunizierenden Druckabnahmezugang (54) aufweist.

4. Vorrichtung (30) nach einem der vorhergehenden Ansprüche, **dadurch gekennzeichnet, dass** das Druckabnehmerzwischenstück (32) über eine flexible Druckabnahmeleitung (42) zur Weiterleitung der abgenommenen Druckänderungen mit der Steuereinrichtung (34) druckverbunden ist.

5. Vorrichtung (30) nach Anspruch 4, **dadurch gekennzeichnet, dass** in der Druckabnahmeleitung (42) ein Filterelement (44), insbesondere zur Aufnahme von Flüssigkeit, vorgesehen ist.

6. Vorrichtung (30) nach einem der vorhergehenden Ansprüche, **dadurch gekennzeichnet, dass** die Steuereinrichtung (34) ein Gehäuse (36) aufweist, und dass das Gehäuse (36) einen mit dem Gehäuse (36) verbundenen Steckverbinder (38) zur Herstellung einer Kommunikationsverbindung mit der Datenverarbeitungsanlage (62) aufweist.

7. Vorrichtung (30) nach einem der vorhergehenden Ansprüche, **dadurch gekennzeichnet, dass** in der Steuereinrichtung (34) das Unterhaltungsprogramm hinterlegt ist oder hinterlegbar ist.

8. Vorrichtung (30) nach einem der vorhergehenden Ansprüche zur Verwendung in der Behandlung und/oder Linderung von Krankheiten.

9. Vorrichtung (30) nach Ansprch 8, wobei die Krankheiten ausgewählt sind aus Erkrankungen der Bronchien und des Nasen-Rachen-Raumes, chronische oder akute Atemwegserkrankungen sowie schweren viralen oder bakteriellen Infektionen der Atemwege.

10. Vorrichtung (30) nach einem der Ansprüche 8 oder 9, wobei die Krankheiten ausgewählt sind aus Bronchitis, Asthma, Zöliakie, COPD (Chronisch-obstruktive Lungenerkrankung), allergischem Schnupfen (Rhinitis allergica), Lungenentzündung, Pilzerkrankungen der Lunge, Lungentuberkulose oder schwerem akuten respiratorischen Syndrom (Sars);
oder
wobei die Krankheit eine Stoffwechselerkrankung ist;
oder
wobei die Krankheit ausgewählt ist aus Mukoviszidose, Typ-1-Diabetes und Typ-2-Diabetes.

## Claims

1. Inhalation support apparatus (30) for an inhalation device (10) that has a mouthpiece (12) and an inhalation mixture generator (14) for supplying an inhalation mixture to be inhaled,
comprising
- a pressure sensor adapter (32), which can be arranged between the inhalation mixture generator (14) and the mouthpiece (12),
wherein the pressure sensor adapter (32) is free of electronic components or free of current-carrying or voltage-carrying components, and pressure changes of the inhalation mixture can be detected by the pressure sensor adapter (32),
- and a control device (34) for controlling an entertainment program in the manner of a computer game running on a data processing installation (62), wherein the control device (34) is configured to control the entertainment program in the manner of a computer game as a function of the pressure changes;
**characterized in that** the pressure sensor adapter (32) can be arranged releasably between the inhalation mixture generator (14) and the mouthpiece (12), in particular being able to be plugged between them.

2. Apparatus (30) according to Claim 1, **characterized in that** the control device (34) is pressure-connected to the pressure sensor adapter (32) for feeding the detected pressure changes to the control device (34).

3. Apparatus (30) according to Claim 1 or 2, **characterized in that** the pressure sensor adapter (32) has a through-flow channel (52) for passage of the inhalation mixture, and a pressure sensor port (54) communicating in terms of pressure with the through-flow channel (52).

4. Apparatus (30) according to one of the preceding claims, **characterized in that** the pressure sensor adapter (32) is pressure-connected to the control device (34) via a flexible pressure sensor line (42) for forwarding the detected pressure changes.

5. Apparatus (30) according to Claim 4, **characterized in that** a filter element (44), in particular for receiving liquid, is provided in the pressure sensor line (42).

6. Apparatus (30) according to one of the preceding claims, **characterized in that** the control device (34) has a housing (36), and **in that** the housing (36) has a plug connector (38), connected to the housing (36), for the purpose of establishing a communication link with the data processing installation (62).

7. Apparatus (30) according to one of the preceding claims, **characterized in that** the entertainment program is stored or can be stored in the control device (34).

8. Apparatus (30) according to one of the preceding claims for use in the treatment and/or alleviation of diseases.

9. Apparatus (30) according to Claim 8, wherein the diseases are chosen from diseases of the bronchi and of the nasopharyngeal space, chronic or acute respiratory tract diseases, and severe viral or bacterial infections of the airways.

10. Apparatus (30) according to either of Claim 8 and 9, wherein the diseases are chosen from bronchitis, asthma, celiac disease, COPD (chronic obstructive pulmonary disease), allergic rhinitis, pneumonia, fungal diseases of the lungs, pulmonary tuberculosis, or severe acute respiratory syndrome (SARS);
or
wherein the disease is a metabolic disorder;
or
wherein the disease is chosen from cystic fibrosis, type 1 diabetes and type 2 diabetes.

## Revendications

1. Dispositif d'aide à l'inhalation (30) pour un dispositif d'inhalation (10) présentant un embout (12) et un générateur de mélange d'inhalation (14) pour fournir un mélange d'inhalation à inhaler, comprenant
- une pièce intermédiaire de réduction de pression (32) qui peut être disposée entre le générateur de mélange d'inhalation (14) et l'embout (12),
la pièce intermédiaire de réduction de pression (32) étant réalisée sans extrémité de composants électroniques ou sans composants conduisant le courant ou la tension, et des variations de pression du mélange d'inhalation pouvant être réduites au moyen de la pièce intermédiaire de réduction de pression (32),
- ainsi qu'un dispositif de commande (34) pour commander un programme de divertissement exécuté sur une installation de traitement de données (62), du type jeu sur ordinateur, le dispositif de commande (34) étant configuré pour commander le programme de divertissement du type jeu sur ordinateur en fonction des variations de pression ;
**caractérisé en ce que** la pièce intermédiaire de réduction de pression (32) peut être disposée de manière amovible, en particulier enfichée entre le générateur de mélange d'inhalation (14) et l'embout (12).

2. Dispositif (30) selon la revendication 1, **caractérisé en ce que** le dispositif de commande (34) est connecté par pression à la pièce intermédiaire de réduction de pression (32) pour transmettre les variations de pression réduites au dispositif de commande (34).

3. Dispositif (30) selon la revendication 1 ou 2, **caractérisé en ce que** la pièce intermédiaire de réduction de pression (32) présente un canal d'écoulement (52) pour conduire le mélange d'inhalation et un accès de réduction de pression (54) communiquant par pression avec le canal d'écoulement (52) .

4. Dispositif (30) selon l'une quelconque des revendications précédentes, **caractérisé en ce que** la pièce intermédiaire de réduction de pression (32) est connectée par pression au dispositif de commande (34) par le biais d'une conduite de réduction de pression flexible (42) de manière à transférer les variations de pression réduites.

5. Dispositif (30) selon la revendication 4, **caractérisé en ce qu'**un élément de filtre (44), en particulier pour recevoir un liquide, est prévu dans la conduite de réduction de pression (42).

6. Dispositif (30) selon l'une quelconque des revendications précédentes, **caractérisé en ce que** le dispositif de commande (34) présente un boîtier (36) et **en ce que** le boîtier (36) présente un connecteur enfichable (38) connecté au boîtier (36) pour établir une connexion de communication avec l'installation de traitement de données (62) .

7. Dispositif (30) selon l'une quelconque des revendications précédentes, **caractérisé en ce que** le programme de divertissement est consigné ou peut être consigné dans le dispositif de commande (34).

8. Dispositif (30) selon l'une quelconque des revendications précédentes, destiné à l'utilisation dans le traitement et/ou le soulagement de maladies.

9. Dispositif (30) selon la revendication 8, les maladies étant choisies parmi les maladies des bronches et de la région rhino-pharyngée, les maladies des voies respiratoires chroniques ou aiguës ainsi que les infections virales ou bactériennes graves des voies respiratoires.

10. Dispositif (30) selon l'une quelconque des revendications 8 ou 9, les maladies étant choisies parmi la bronchite, l'asthme, la maladie coeliaque, la bronchopneumopathie chronique obstructive (BPCO), le rhume allergique (Rhinitis allergica), les inflammations des poumons, les mycoses des poumons, la tuberculose ou le syndrome respiratoire aigu grave (Sars) ; ou
la maladie étant un trouble du métabolisme ; ou
la maladie étant choisie parmi la mucoviscidose, le diabète de type 1 et le diabète de type 2.
